(19) Europäisches Patentamt
European Patent Office
Office européen des brevets

(11) Publication number: 0 088 831 A1

(12) EUROPEAN PATENT APPLICATION

(21) Application number: 82200327.3

(22) Date of filing: 11.03.82

(51) Int. Cl.³: C 07 C 148/00
C 07 C 149/30, C 07 D 501/36
C 07 D 285/12, C 07 D 257/04
C 07 C 149/31, C 07 C 149/34
C 07 C 149/40, C 07 C 149/415
C 07 C 153/11, C 07 D 233/84

(43) Date of publication of application:
21.09.83 Bulletin 83/38

(84) Designated Contracting States:
AT BE CH DE FR GB IT LI LU NL SE

(71) Applicant: Gist - Brocades N.V.
Wateringseweg 1 P.O. Box 1
NL-2600 MA Delft(NL)

(72) Inventor: Bruynes, Cornelis Adrianus
Ridderhoflaan 51
NL-2396 CK Koudekerk a/d Rijn(NL)

(72) Inventor: Jurriens, Theodorus Klaas
Van der Haertstraat 43
NL-2613 ZA Delft(NL)

(74) Representative: Broekhuijsen, Robbert et al,
c/o Gist-Brocades N.V. Wateringseweg 1 P.O. Box 1
NL-2600 MA Delft(NL)

# (54) Process for the preparation of thioethers.

(57) Process for the preparation of thioethers by reacting a silylated thiol of the general formula:

$$R - S - SiR_1R_2R_3 \qquad I$$

wherein R represents an organic group and $R_1$, $R_2$ and $R_3$ are the same or different and each represents an alkyl group having 1 to 4 carbon atoms, with an organic halide, sulphate or sulphonate in the presence of hexamethylphosphoric triamide as a solvent or co-solvent. The process is carried out under neutral conditions in aprotic solvents at a temperature between 0° and 150°C.

EP 0 088 831 A1

Croydon Printing Company Ltd.

Process for the preparation of thioethers

The invention relates to a new process for the preparation of thioethers.

Several methods for the preparation of thioethers are known; see for example, S. Oae, Organic Chemistry of Sulfur, Plenum Press, New York, 1977, chapter 6.

In one method starting with thiols these are first converted to the corresponding mercaptides, usually by reaction with aqueous or alcoholic solutions of sodium hydroxide or sodium ethoxide. The mercaptides are then reacted with organic halides, dialkyl sulphates or alkyl sulphonates. Accordingly, these reactions are carried out under alkaline conditions in protic solvents, which may lead to undesired side-reactions since other functionalites present in the reactants can be effected as well.

In another method starting with thiols these are first converted to their trimethylsilyl derivatives by replacing the hydrogen atom of the mercapto group by a trimethylsilyl group.

European patent application No. 81.200981.9, which is not pre-published, discloses a new method for the introduction of a thio-substituent in the 3-methyl group of cephalosporanic acid derivatives by reacting a 7-acylamino-3-bromomethyl-3-cephem-4-carboxylic acid 1-oxide derivative with a trimethylsilylated thiol. These reactions proceed smoothly and with virtually quantitative yields.

The method for preparing thioethers from alkylthio(trimethyl)silanes and alkyl halides requires, however, high temperatures and reaction times of up to several weeks to obtain low to moderate yields (E.W. Abel, J.Chem.Soc., 4406 (1960); E.W. Abel, D.A. Armitage and R.P. Bush, J.Chem.Soc., 2455 (1964)). Similarly, the reaction of arylthio(trimethyl)silanes with methyl iodide is reported to be sluggish in the presence of a solvent, even at high temperatures, and does not give the anticipated thioether in a satisfactory yield. The same holds for the reaction with benzyl bromide. Only with the very reactive phenacyl bromide a good yield of phenacyl phenyl

sulphide could be obtained by heating at 60°C for 5 hours (S. Kozuka, T. Higashino and T. Kitamura, J.Chem.Soc. Japan, 54, 1420 (1981)).

It has now surprisingly been found that reactions of trialkylsilylated thiols with an organic halide, sulphate or sulphonate proceed under mild conditions, in relatively short times and give good to excellent yields of the thioethers when the reactions are carried out in the presence of hexamethylphosphoric triamide as a solvent or a co-solvent.

Thus, the invention provides a new process for the preparation of thioethers by reacting a silylated thiol of the general formula:

$$R - S - SiR_1R_2R_3 \qquad I$$

wherein R represents an organic group and $R_1$, $R_2$ and $R_3$ are the same or different and each represents an alkyl group having 1 to 4 carbon atoms, with an organic halide, sulphate or sulphonate in the presence of hexamethylphosphoric triamide as a solvent or a co-solvent.

The process of the invention is carried out under neutral conditions in aprotic solvents, thus preventing any side-reactions as may take place under the alkaline conditions prevailing in one general method of the prior art. Suitable solvents are, for example, acetonitrile, dichloromethane, toluene and ethyl acetate.

The process may be carried out at relatively low temperatures as compared with the prior art processes, generally at a temperature between 0° and 150°C, preferably between 20° and 80°C.

The application of hexamethylphosphoric triamide constitutes a considerable improvement over the prior art in the preparation of thioethers from trimethylsilylated thiols. The process of the present invention may also advantageously be used in the preparation of 7-acylamino-3-(thio-substituted)methyl-3-cephem-4-carboxylic acid 1-oxide derivatives from the corresponding 3-bromomethyl derivatives, viz. in those cases in which the trimethylsilylated thiol is of very low reactivity or when it is desirable to lower the reaction

BAD ORIGINAL

temperature or to shorten the reaction time.

The organic halide used in the process of the invention can be a chloride, bromide or iodide, but preferably is a bromide or iodide. Various types of organic halides are suitable. Examples are halides wherein the organic group is a straight or branched-chain alkyl or alkenyl group, an aryl or aralkyl group or a heterocyclic group. Each of these groups may be further substituted by one or more groups which do not interfere with the reaction between the halides and the silylated thiols of formula I.

Suitable substituents are, for example, halogen atoms, alkyl and alkylthio groups, the nitro and the cyano group and esterified carboxyl groups. Examples of suitable halides for use in the process of the invention are halides wherein the organic group is a methyl, ethyl, isopropyl, butyl, allyl, phenyl or benzyl group, each of which may be substituted as indicated herebefore.

A particularly important group of organic halides is formed by 7-acylamino-3-bromomethyl-3-cephem-4-carboxylic acid 1-oxide derivatives which are valuable intermediates in methods for the preparation of therapeutically active cephalosporins.

When two halogen atoms are present in the molecule of an organic halide, dithioethers may be formed. This is particularly the case when the organic dihalide is a dihalogenomethane and, to a lesser extent, when it is a 1,2-dihalogenoethane. It is known from the literature that the product formed by replacing one of the halogen atoms of a dihalogenomethane by a thio-substituent results in a product having a very high tendency to nucleophilic substitution. So it is known that even in the presence of an excess of dichloromethane the reaction of sodium p-chlorothiophenolate with dichloromethane results in the formation of only the di-substitution product di-(p-chlorophenylthio)methane (A.M. Kuliev, E.N. Usubova, Yu.M. Sultanov and A.B. Kuliev, Azerb. Khim. Zh. 46 (1966)). Similar phenomena were noticed when applying the method of the present invention.

Suitable organic sulphates and sulphonates for use in the process of the invention are dialkyl sulphates and alkyl sulphonates, especially the alkyl esters of benzene and

naphthalene sulphonic acids.

Various types of silylated thiols according to formula I may be used in the process of the invention. For example, the organic group R may be an alkyl, aryl, aralkyl or heterocyclic group. Each of these groups may be further substituted by one or more groups as specified above for the organic halides.

Suitable alkyl groups are straight or branched-chain alkyl groups; a suitable aryl group is, for example, the phenyl group. Suitable heterocyclic groups are, for example, 5- or 6-membered heterocyclic groups having one or more nitrogen or sulphur atoms as the heteroatoms. The nitrogen-heterocyclic trialkylsilylated thiols yield mainly the S-substitution products on reaction with halides, but N-substitution also was noticed.

When the organic group R contains a carboxyl group, this group will be silylated as well during the process of preparing the silylated thiols of general formula I from the corresponding thiols. Thus, when such a silylated thiol is reacted according to the process of the invention with an organic halide, sulphate or sulphonate, the carboxyl group is protected against undesired side-reactions. After the reaction has been completed any silyloxycarbonyl groups present in the product, if desired, can easily be converted to free carboxyl groups by known methods.

Likewise, when any free carboxyl groups are present in the organic halides used as starting materials in the process of the invention, these carboxyl groups can be protected with a silyl group before this halide is reacted with the silylated thiol.

The trialkylsilylated thiols of formula I can be prepared by any known method. Trimethylsilylated thiols are preferably prepared by the method disclosed in European patent application 81.200771.4 in which thiols are trimethylsilylated with 1,1,1,3,3,3-hexamethyldisilazane in the presence of a catalyst. The trimethylsilylated thiol can be isolated from the reaction mixture obtained but, more conveniently, the reaction mixture itself also can be used as a starting material in the process of the present invention.

BAD ORIGINAL

The invention is further illustrated with the following examples in which

1) PMR spectra were recorded at 60 MHz unless otherwise stated; chemical shifts are reported relative to tetramethylsilane ($\delta$=0) used as an internal standard.

2) $^{13}C$ NMR spectra were taken at 20 MHz; tetramethylsilane ($\delta$=0) was used as an internal standard.

3) IR spectra were obtained on KBr discs.

4) Boiling points and melting points are uncorrected.

5) Quantitative HPLC analyses were performed with solutions of appropriate concentrations which were prepared by standard techniques. Whenever required, the purity of the reference substance was determined by means of quantitative PMR analysis using an internal standard technique. The accuracy is estimated to be 5%.

6) Reactions were carried out in a dry nitrogen atmosphere. A stream of nitrogen was led over the reaction mixture and, in case of catalysed silylations with hexamethyldisilazane, this nitrogen was passed into water and used to determine the reaction time by titrating the ammonia generated in the reaction with 0.1 or 1.0 N sulphuric acid, whichever was appropriate. Other reactions were followed by thin-layer chromatography on silicagel G.

7) Solvents used were dried over 4A molecular sieves and were of an alcohol-free grade. Solutions were dried over magnesium sulphate.

8) All evaporations were performed under reduced pressure on a rotary evaporator at a bath temperature not exceeding 35°C.

## Example 1

To a solution of 192 mg (1.12 mmole) of benzyl bromide in 1 ml of acetonitrile and 0.36 ml of hexamethylphosphoric triamide, a solution of 171 mg (0.94 mmole) of phenylthio(trimethyl)silane in 1 ml of acetonitrile was added and the mixture was stirred for 1.5 hour at 50°C. Then, 2 ml of methanol were added. According to quantitative HPLC analysis the solution thus obtained contained 183 mg of benzyl phenyl sulphide, which amounts to a yield of 97%. The product has b.p. 188-192°C/22 mm Hg and m.p. 39-40°C.

## Example 2

(a) Benzyl bromide (0.60 ml; 5.0 mmoles) was added at 50°C to a solution of 816 mg (3.64 mmoles) of phenylthio(triethyl)silane in 5 ml of acetonitrile and 0.9 ml of hexamethylphosphoric triamide. After stirring for 45 minutes at 50°C the reaction was quenched with 2 ml of ethanol. HPLC analysis of the diluted reaction mixture revealed the yield of benzyl phenyl sulphide to be 96%.

(b) Reaction of 949 mg (4.24 mmoles) of phenylthio(dimethyl)(t-butyl)silane with the same reagents and at the same temperature as mentioned under (a) for 15 minutes yielded 90% of benzyl phenyl sulphide.

## Example 3

A mixture of 11.52 g (63 mmoles) of phenylthio(trimethyl)silane, 22 ml of hexamethylphosphoric triamide and 18.8 ml (250 mmoles) of ethyl bromide was heated in a bath of 40°C for 2 hours after which the conversion was complete. A solid had been formed in the reaction mixture. Heating was continued for 1 hour and then the mixture was poured into a mixture of 150 ml of ethyl acetate and 50 ml of water. The ethyl acetate layer was separated and washed, subsequently twice with 50 ml of water, 50 ml of a saturated sodium bicarbonate solution and twice with 25 ml of water. The combined water layers were

extracted three times with 25 ml of ethyl acetate. The combined extracts were washed twice with 25 ml of water, dried and evaporated. The residue was vacuum distillated to yield 7.25 g (83%) of ethyl phenyl sulphide, b.p. 81.5- 84°C/14 mm Hg, $n_D^{25}$ 1.5632.

Example 4

A solution of 11.34 g (57 mmoles) of phenacyl bromide in 15 ml of acetonitrile was added to a solution of 9.51 g (52.2 mmoles) of phenylthio(trimethyl)silane in 5 ml of acetonitrile and 9.8 ml of hexamethylphosphoric triamide, by which the temperature rose from 20 to 75°C. The reaction was complete within 5 minutes. By means of quantitative HPLC analysis it was found that the yield of phenacyl phenyl sulphide was quantitative. The reaction mixture was worked up as described in Example 3, but the residue was crystallized from ethanol, yielding 91% of phenacyl phenyl sulphide, m.p. 51-52°C.

Example 5

Phenacyl bromide (6.70 g; 33.6 mmoles) was added to a mixture of 4.96 g (30.6 mmoles) of butylthio(trimethyl)-silane, 6.0 ml of acetonitrile and 5.9 ml of hexamethylphosphoric triamide. After stirring for 1.5 hour at 80°C, the mixture was cooled and subjected to quantitative HPLC analysis. It was found that 92% of butyl phenacyl sulphide had been formed.

Example 6

Methyl iodide (4.80 ml; 77 mmoles) was added to a solution of 12.87 g (70.7 mmoles) of phenylthio(trimethyl)-silane in 15 ml of acetonitrile and 13.5 ml of hexamethylphosphoric triamide, which caused the temperature to rise from room temperature to 90°C. After stirring for 0.5 hour the reaction mixture was cooled to room temperature and a sample

was taken for quantitative HPLC analysis, by which it was found that the yield of thioanisole was 98%. The acetonitrile was evaporated and the residue was treated as described in Example 3. Vacuum distillation of the residue yielded 7.89 g (90%) of thioanisole, b.p. 89-92°C/18-22 mm Hg, $n_D^{25}$ 1.5834.

Example 7

Methyl p-toluenesulphonate (3.08 g; 16.6 mmoles) was added to a solution of 1.90 g (10.4 mmoles) of phenylthio(trimethyl)silane in a mixture of 4 ml of acetonitrile and 2 ml of hexamethylphosphoric triamide. After stirring for 1 hour at 70°C the mixture was cooled and subjected to quantitative HPLC analysis. The yield of thioanisole was found to be 94%.

Example 8

Dimethyl sulphate (1.9 ml; 20 mmoles) was added to a solution of 3.38 g (18.6 mmoles) of phenylthio(trimethyl)-silane in a mixture of 7 ml of acetonitrile and 2.5 ml of hexamethylphosphoric triamide. After stirring for 2.5 hours at ambient temperature a quantitative HPLC analysis was carried out by which it was found that the yield of thioanisole was 75%.

Example 9

To a solution of 0.91 g (5.0 mmoles) of phenylthio-(trimethyl)silane in 25 ml of dry acetonitrile, 1 ml of hexamethylphosphoric triamide and 1.0 g (4.15 mmoles) of 3-bromo-4-hydroxycoumarin were added. After refluxing for 1 hour the mixture was evaporated. The residue was dissolved in 125 ml of ethyl acetate and the solution obtained was washed twice with 125 ml of water. The ethyl acetate layer was dried and evaporated. The solid residue was washed with 100 ml of heptane and then with three portions of 50% ethanol. The product was vacuum dried at 40°C. There was obtained 0.79 g (70.5%) of 3-phenylthio-4-hydroxycoumarin, m.p. 186-189°C.

BAD ORIGINAL BAD ORIGINAL

Example 10

A mixture consisting of 11.51 g (63.2 mmoles) of phenylthio(trimethyl)silane, 5.0 ml (77 mmoles) of bromochloromethane, 12 ml of acetonitrile and 11 ml of hexamethylphosphoric triamide was heated for 1 hour in a bath of 80°C. Then it was poured into 100 ml of ethyl acetate and the solution obtained was washed three times with 10 ml of water, three times with 10 ml of 1N KOH solution and finally three times with 10 ml of water. The solution was dried and evaporated. After addition of 25 ml of carbon tetrachloride, the evaporation was repeated. There were obtained 6.95 g (94.8%) of di(phenylthio)methane, m.p. 34-36°C.

Example 11

To a mixture of 1.36 g (6.3 mmoles) of 4-chlorophenylthio(trimethyl)silane, 20 ml of acetonitrile and 2.3 ml of hexamethylphosphoric triamide, 1.50 g (7.0 mmoles) of 4-nitrobenzyl bromide was added. The conversion was complete after stirring for 5 minutes at room temperature. The reaction mixture was evaporated to dryness and the residue was dissolved in 50 ml of ethyl acetate. The solution was washed with subsequently, water (twice 10 ml), 10 ml of a 1N KOH solution and water (twice 10 ml). The ethyl acetate layer was dried and evaporated to dryness. The solid residue was washed with heptane on a sintered glass filter. The crystals obtained were vacuum dried at room temperature. There was obtained 1.67 g (95%) of 4-chlorophenyl 4-nitrobenzyl sulphide, m.p. 66-68°C.

Example 12

A mixture consisting of 12.21 g (56.5 mmoles) of 4-chlorophenylthio(trimethyl)silane, 20 ml of hexamethylphosphoric triamide and 18.7 ml (226 mmoles) of 1-bromo-2-chloroethane was heated at 60°C. It was established that the conversion was complete after 10 minutes. After treating the reaction mixture as described in Example 3 there were obtained

10.03 g (85.7%) of 2-chloroethyl 4-chlorophenyl sulphide, b.p. 105- 109°C/1.0 mm Hg, m.p. 28-29°C. The residue of the distillation contained 1,2-bis(4-chlorophenylthio)ethane as a by-product, which was crystallized from methanol, m.p. 87-88°C.

## Example 13

To a mixture of 10.04 g (51.2 mmoles) of p-tolylthio(trimethyl)silane in 20 ml of acetonitrile and 18 ml of hexamethylphosphoric triamide, 5.1 ml (59 mmoles) of allyl bromide were added. The conversion was complete after stirring for 20 minutes at room temperature. The reaction mixture was treated as described in Example 3. There were obtained 7.1 g (85%) of allyl p-tolyl sulphide, b.p. 110-111°C/14 mm Hg, $n_D^{25}$ 1.5644.

## Example 14

Trimethylsilyl bromoacetate (5.78 g; 27.5 mmoles) was added to a solution of 4.85 g (25 mmoles) of p-tolylthio-(trimethyl)silane in 10 ml of acetonitrile and 5 ml of hexamethylphosphoric triamide. This caused a rise in temperature from 22 to 69°C within 2 minutes. According to thin layer chromatography the reaction was complete within 5 minutes. After evaporation of the acetonitrile, 100 ml of water were added and the solution obtained was extracted with three 50 ml portions of ethyl acetate. The combined extracts were washed with 0.1 N HCl, dried, filtered and evaporated to dryness. The residue was crystallized from 30 ml of a 1:5 mixture of toluene and hexane to yield 4.08 g (90%) of p-tolylthioacetic acid, m.p. 94.5-95.0°C. Work-up of the mother liquor yielded 0.31 g (6.8%) of material with m.p. 86-89°C.

## Example 15

(a) 3,4-Dichlorophenylthio(trimethyl)silane was prepared by refluxing a mixture of 8.45 g (47 mmoles) of 3,4-dichlorothiophenol, 22 mg (0.045 mmole) of tetraphenyl imidodiphosphate, 10 ml of 1,2-dichloroethane and 7.4 ml (35 mmoles) of

BAD ORIGINAL

hexamethyldisilazane for 45 minutes. The product was isolated by distillation; yield 95.3%, b.p. 96-97°C/0.8 mm Hg, $n_D^{25}$ 1.5600.

(b) Chloroacetonitrile (3.5 ml; 55 mmoles) was added to a refluxing solution of 11.0 g (44 mmoles) of 3,4-dichlorophenylthio(trimethyl)silane in a mixture of 25 ml of acetonitrile and 10 ml of hexamethylphosphoric triamide. The reaction had run to completion within 2 minutes. The residue obtained after evaporation of the acetonitrile was dissolved in ethyl acetate, washed with water and dried. Distillation yielded 9.30 g (93%) of (3,4-dichlorophenylthio)acetonitrile, b.p. 127-130°C/0.4 mm Hg, $n_D^{25}$ 1.5920.

## Example 16

4-Nitrobenzyl bromide (4.62 g; 21.3 mmoles) was added to a solution of 4.60 g (19.4 mmoles) of trimethylsilyl trimethylsilylthioacetate in 10 ml of acetonitrile and 4.9 ml of hexamethylphosphoric triamide. The reaction had run to completion after stirring for 30 minutes at 80°C. The reaction mixture was cooled, diluted and subjected to quantitative HPLC analysis. It was found that 97% of 4-nitrobenzylthioacetic acid had been formed.

## Example 17

(a) To a refluxing mixture of 9.9 g (75 mmoles) of 5-mercapto-2-methyl-1,3,4-thiadiazole, 27 mg (0.15 mmole) of saccharin, 50 ml of acetonitrile and 20 ml of hexamethylphosphoric triamide, 15 ml (72 mmoles) of hexamethyldisilazane were added. After refluxing for 4.5 hours the calculated amount of ammonia had been evolved. Refluxing was continued for 0.5 hour; the mixture (containing 5-trimethylsilylthio-2-methyl-1,3,4-thiadiazole) was cooled to room temperature and 9.9 ml (80 mmoles) of benzyl bromide were added. This raised the temperature to about 50°C. All starting material was consumed within 5 minutes. The acetonitrile was removed by evaporation; 100 ml of ethyl acetate were added to the residue

and then it was poured out into 250 ml of water. The layers were separated and the water layer was extracted three times with 30 ml of ethyl acetate. The combined extracts were washed with a 10% NaCl solution, dried, filtered and evaporated to dryness. The solid residue was washed with hexane containing some diethyl ether. There were obtained 15.91 g (96.4%) of 5-benzylthio-2-methyl-1,3,4-thiadiazole, m.p. 60-63°C. Crystallization from a mixture of ethyl acetate and petroleum ether (boiling range 40-60°C) provided 14.27 g of the pure substance, m.p. 62.5- 63.5°C.

UV ($CH_3CN$): $\lambda_{max}$ 267 nm ($\epsilon$=6250).

This product was used as the reference substance for the HPLC analysis mentioned herebelow and in Example 18.

(b) To a solution of 264 mg (2 mmoles) of 5-mercapto-2-methyl-1,3,4-thiadiazole in 5 ml of toluene and 1.06 ml (6.1 mmoles) of hexamethylphosphoric triamide, 2.0 mg (0.004 mmole) of di-4-nitrophenyl N-(4-toluenesulphonyl)phosphoramidate were added and subsequently, while refluxing, 0.50 ml (2.4 mmoles) of hexamethyldisilazane. After refluxing had been continued for 1.5 hour, the solution was evaporated to dryness. The residue was dissolved in 4 ml of acetonitrile and to the solution thus obtained 0.28 ml (2.3 mmoles) of benzyl bromide was added. It was established that the conversion was complete after stirring for 10 minutes. HPLC analysis of the reaction mixture indicated a quantitative yield of 5-benzylthio-2-methyl-1,3,4-thiadiazole.

## Example 18

(a) 13.2 mg (100 mmoles) of 5-mercapto-2-methyl-1,3,4-thiadiazole were converted into 5-trimethylsilylthio-2-methyl-1,3,4-thiadiazole by refluxing for 1.5 hour in 75 ml of toluene with 15 ml (72 mmoles) of hexamethyldisilazane, using 50 mg (0.1 mmole) of di-4-nitrophenyl N-(4-toluenesulphonyl)-phosphoramidate as a catalyst. The solvent and the excess of hexamethyldisilazane were evaporated and the residue was dissolved in dry hexamethylphosphoric triamide.

(b) To 1 ml of this solution, which according to HPLC

BAD ORIGINAL

analysis contained 1.23 mmole of 5-trimethylsilylthio-2-methyl-1,3,4-thiadiazole, 3 ml of acetonitrile and 0.20 ml (1.68 mmole) of benzyl bromide were added. The mixture was stirred for 0.5 hour at room temperature after which, according to HPLC analysis, 1.28 mmole (104%) of 5-benzylthio-2-methyl-1,3,4-thiadiazole had been formed.

(c) To 1 ml of the solution prepared under (a) 3 ml of acetonitrile and 0.20 ml (1.74 mmole) of benzyl chloride were added. After stirring for 1.5 hour at 65°C, 1.15 mmole (93.5%) of 5-benzylthio-2-methyl-1,3,4-thiadiazole had been formed, according to HPLC analysis. There was still 8% of the starting material present in the reaction mixture.

## Example 19

A mixture consisting of 13.2 g (0.10 mole) of 5-mercapto-2-methyl-1,3,4-thiadiazole, 20 ml of toluene, 25 ml of hexamethylphosphoric triamide, 20 ml (0.096 mole) of hexamethyldisilazane and 50 mg (0.1 mmole) of di-4-nitrophenyl N-(4-toluenesulphonyl)phosphoramidate was refluxed for 90 minutes. The mixture was then concentrated and to the solution of 5-trimethylsilylthio-2-methyl-1,3,4-thiadiazole in hexamethylphosphoric triamide thus obtained, 15 ml of acetonitrile and 21.5 ml (0.20 mole) of butyl bromide were added. The mixture was stirred for 5 hours at room temperature and then concentrated. Water (100 ml) and sodium bicarbonate were added till the evolution of carbon dioxide stopped. The mixture was extracted with ethyl acetate and the extract was dried, filtered and evaporated to dryness. The residue was separated by chromatography (silica gel; dichloromethane and a 95:5 mixture of dichloromethane and acetone, respectively). There were obtained 0.4 g of 3-butyl-5-methyl-$\Delta^4$-1,3,4-thiadiazoline-2-thione as a by-product.

PMR ($CCl_4$): 0.8 - 2.0 (m, 7H); 2.39 (s, 3H); 4.15 (t, 2H, J 7.2 Hz).

$^{13}C$-NMR ($CDCl_3$): 13.4; 15.9; 19.5; 29.7; 50.4; 155.3; 185.7.

and 10.9 g (58%) of 5-butylthio-2-methyl-1,3,4-thiadiazole,

b.p. 97-99°C/0.6 mm Hg, $n^{25}$ 1.5492.

UV ($CH_3CN$):$\lambda_{max}$ 266 nm ($\varepsilon$ = 7000).

PMR ($CDCl_3$): 0.8 - 2.0 (m, 7H); 2.70 (s, 3H); 3.27 (t, 2H, J 7.4 Hz).

13C-NMR ($CDCl_3$): 13.6; 15.1; 21.4; 30.8; 33.5; 164.2; 165.4.

## Example 20

(a) In the manner described in Example 19, 1.32 g (10 mmoles) of 5-mercapto-2-methyl-1,3,4-thiadiazole was silylated with 2.6 ml (15 mmoles) of hexamethyldisilazane in 10 ml of toluene and 2.6 ml of hexamethylphosphoric triamide, using 5 mg (0.027 mmole) of saccharin as a catalyst, by refluxing for 1.5 hour. After concentration by evaporation, 5 ml of acetonitrile were added. The solution obtained was heated at 50°C and 2.1 ml (20 mmoles) of butyl bromide were added. The conversion was complete after stirring for 3 hours at this temperature. According to quantitative HPLC analysis there had been formed 8.91 mmoles (89.1%) of 5-butylthio-2-methyl-1,3,4-thiadiazole, while 5.3% of 3-butyl-5-methyl-$\Delta^4$-1,3,4-thiadiazoline-2-thione was found as a by-product.

(b) The experiment was repeated, but now instead of butyl bromide, 2.3 ml (20 mmoles) of butyl iodide were added while stirring was effected at room temperature. The conversion was complete after 3 hours. By means of quantitative HPLC analysis it was established that the yield of 5-butylthio-2-methyl-1,3,4-thiadiazole was 89.1%, while less than 1.3% of said by-product had been formed.

## Example 21

Chloromethyl methyl sulphide (1.85 ml; 22 mmoles) was added to a refluxing solution of 4.18 g (20.5 mmoles) of 5-trimethylsilylthio-2-methyl-1,3,4-thiadiazole in a mixture of 15 ml of acetonitrile and 3.85 ml of hexamethylphosphoric triamide. After refluxing for 10 minutes the reaction was complete. Ethanol (5 ml) was added and the mixture was

evaporated to dryness. The residue was dissolved in ethyl acetate and the solution obtained was extracted with diluted sodium hydroxide solution (pH 10) and with water. The ethyl acetate layer was dried and evaporated. The residue contained two components which were separated by chromatography over silica gel, using a 10% solution of acetone in dichloromethane as an eluent. From the first fractions 0.23 g (5.8%) of 3-methylthiomethyl-5-methyl- $\Delta^4$-1,3,4-thiadiazoline-2-thione was isolated as a by-product.

PMR ($CDCl_3$): 2.34 (s, 3H); 2.50 (s, 3H); 5.35 (s, 2H).

From the following fractions 3.60 g (91.4%) of 5-(methylthio)methylthio-2-methyl-1,3,4-thiadiazole were isolated, b.p. 115-116°C/0.4 mm Hg, $n_D^{25}$ 1.6222.

PMR ($CDCl_3$): 2.29 (s, 3H); 2.76 (s, 3H); 4.41 (s, 2H).

## Example 22

5-Trimethylsilylthio-2-methyl-1,3,4-thiadiazole (TMT) was reacted with allyl chloride and with allyl bromide under various conditions in the presence of hexamethylphosphoric triamide (HMPT). Yields of 5-allylthio-2-methyl-1,3,4-thiadiazole were determined by HPLC analysis. The results are summarized in the following table.

| TMT (mmoles) | $CH_3CH=CH_2X$ (mmoles) | HMPT (ml) | solvent (10 ml) | temperature (°C) | reaction time (min.) | yield (%) |
|---|---|---|---|---|---|---|
| 7.2 | 8.5 (X=Br) | 1.5 | $CH_3CN$ | 20 | 90 | 99 |
| 9.7 | 11.6 (X=Br) | 2.0 | $CH_3CN$ | 45 | 45 | 100 |
| 7.7 | 9.1 (X=Br) | 1.6 | $CH_2Cl_2$ | 45 | 90 | 97 |
| 9.3 | 10.8 (X=Cl) | 1.9 | $CH_3CN$ | 45 | 240 | 93 |

BAD ORIGINAL

The reference compound was isolated by distillation from a run without HMPT, b.p. 102°C/1.0 mm Hg.

UV ($CH_3CN$): $\lambda_{max}$ 265 nm ($\varepsilon$ = 6500).

PMR ($CDCl_3$): 2.65 (s, 3H), 3.83 and 3.94 (d, 2H), 5.05 - 6.35 (m, 3H).

## Example 23

To a solution of 2.56 g (20 mmoles) of 1-phenyl-5-mercapto-1H-tetrazole in 5 ml of hexamethylphosphoric triamide, 50 ml of hexane and 48 mg (0.1 mmole) of tetraphenyl imidodiphosphate were added. The mixture was refluxed and 8.3 ml (40 mmoles) of hexamethyldisilazane were added, whereupon a precipitate was formed. After addition of 25 ml of ethyl acetate refluxing was continued for 2 hours and then the mixture was evaporated to dryness at 40°C under oil pump vacuum. The 1-phenyl-5-trimethylsilylthio-1H-tetrazole thus obtained was dissolved in 45 ml of acetonitrile. Subsequently, 4.75 g (22 mmoles) of 4-nitrobenzyl bromide were added to the solution which resulted immediately in the forming of a precipitate. After stirring for 15 minutes at room temperature the mixture was evaporated and the residue was dissolved in a mixture of 50 ml of water and 50 ml of ethyl acetate. The ethyl acetate layer was separated, washed three times with 10 ml of water, dried, filtered and evaporated to dryness. The residue was crystallized from 80 ml of a 1:1 mixture of ethyl acetate and heptane. There were obtained 4.23 g (67.6%) of 1-phenyl-5-(4-nitrobenzyl)thio-1H-tetrazole, m.p. 153-154°C.

UV ($CH_3CN$): $\lambda_{max}$ 264 nm ($\varepsilon$ 13.000).

PMR ($CDCl_3$): 4.71 (s, 2H); 7.54 (s, 5H); 7.72 and 8.24 (ABq, 4H, J 9 Hz).

This product was used as the reference substance for the HPLC analysis mentioned in Examples 24 and 25.

## Example 24

264.5 mg (1.49 mmole) of 1-phenyl-5-mercapto-1H-tetrazole were suspended in a mixture of 15 ml of chloroform

BAD ORIGINAL

and 15 ml of 1,2-dichloroethane. There were added 2 mg (0.01 mmole) of saccharin and subsequently, while refluxing, 0.65 ml (3.1 mmoles) of hexamethyldisilazane. After refluxing for 1 hour the mixture was evaporated to dryness and the residue dissolved in 10 ml of acetonitrile and 0.3 ml of hexamethylphosphoric triamide. To the solution obtained, 0.35 g (1.62 mmole) of 4-nitrobenzyl bromide was added, which resulted immediately in the forming of a solid. After stirring for 10 minutes at room temperature quantitative HPLC analysis was carried out. A yield of 95% of 1-phenyl-5-(4-nitrobenzyl)thio-1H-tetrazole was found.

Example 25

In the manner described in Example 23, 2.60 g (14.6 mmoles) of 1-phenyl-5-mercapto-1H-tetrazole were silylated in 1.25 hour with 7.5 ml (36 mmoles) of hexamethyldisilazane in a mixture of 30 ml of chloroform and 30 ml of 1,2-dichloroethane, using 19 mg (0.1 mmole) of saccharin as a catalyst. After evaporation to dryness the residue was dissolved in acetonitrile and 5 ml of this solution, containing 0.72 mmole of 1-phenyl-5-trimethylsilylthio-1H-tetrazole, were added to a solution of 0.17 g (0.79 mmole) of 4-nitrobenzyl bromide in 0.17 ml of hexamethylphosphoric triamide and also to a solution of 0.13 g (0.76 mmole) of 4-nitrobenzyl chloride in 0.17 ml of hexamethylphosphoric triamide. After stirring for 1 hour the reactions were quenched with 1 ml of methanol and the yields of product determined by HPLC analysis. The yields of 1-phenyl-5-(4-nitrobenzyl)thio-1H-tetrazole were 102% and 98%, respectively.

Example 26

(a) Hexamethyldisilazane (3.75 ml; 18 mmoles) was added to a refluxing solution of 2.83 g (24.4 mmoles) of 5-mercapto-1-methyl-1H-tetrazole and 50 mg (0.28 mmole) of saccharin in 30 ml of acetonitrile. After refluxing for 90 minutes the reaction mixture was evaporated to dryness. There was obtained

BAD ORIGINAL

1-methyl-5-trimethylsilylthio-1H-tetrazole as a liquid, $n_D^{25}$ 1.5175 .

PMR ($CCl_4$): 0.81 (s, 9H); 3.79 (s, 3H).

(b) This product was dissolved in a mixture of 10 ml of acetonitrile and 5 ml of hexamethylphosphoric triamide. Isopropyl iodide (2.75 ml; 27.5 mmoles) was added to this solution and the mixture obtained was stirred for 90 minutes at room temperature, after which the acetonitrile was evaporated. Ethyl acetate (25 ml) and water (25 ml) were added to the residue, the pH of which was adjusted to 10.0 with 1N NaOH. The layers were separated and the ethyl acetate layer was washed with sodium hydroxide solution of pH 10.0 (25 ml) and water (25 ml), dried and evaporated. The residue (4.30 g) was further purified by chromatography over silica gel (150 g) using ethyl acetate as an eluent. After evaporation of the appropriate fractions 3.04 g (80%) of 5-isopropylthio-1-methyl-1H-tetrazole, $n_D^{25}$ 1.5030, was obtained.

PMR ($CDCl_3$): 1.50 (d, 6H, J 6.0 Hz); 3.98 (s, 3H); 4.05 (q, 1H, J 6.0 Hz).

Example 27

(a) Phenacyl bromide (1.62 g; 8.1 mmoles) was added to a solution of 1.90 g (7.76 mmoles) of 1-trimethylsilyl-3-trimethylsilylthio-1H-1,2,4-triazole in 10 ml of acetonitrile and 1.55 ml of hexamethylphosphoric triamide. The reaction had run to completion after stirring for 15 minutes at room temperature. The precipitate that formed after the addition of 4 ml of methanol was filtered off and washed with diethyl ether and with hexane. A yield of 1.85 g (79.5%) of 3-phenacylthio-1H-1,2,4-triazole hydrogen bromide salt, m.p. 210.5-202°C (dec.) was obtained. A second crop (0.3 g; 13%; m.p. 191-192°C (dec.)) was isolated from the mother liquor. 0.90 g of this salt was taken up in 10 ml of water which was layered with 20 ml of ethyl acetate. The pH was adjusted to 9 with 1N KOH and the free base was extracted from the water layer with ethyl acetate. In this way there was obtained 0.66 g (100%) of 3-phenacylthio-1H-1,2,4-triazole, m.p. 119-120°C. Crystallization from ethyl acetate raised the melting point to 120-121°C.

PMR (DMSO-d6): 4.87 (s, 2H); 7.4-8.3 (m, 6H); 8.53 (s, 1H).

IR: 3145, 1699, 1660, 1593, 1578, 1485 $cm^{-1}$.

(b) The 1-trimethylsilyl-3-trimethylsilylthio-1H-1,2,4-triazole was prepared in the following way: hexamethyldisilazane (29.2 ml; 140 mmoles) was added to a refluxing suspension of 9.70 g (96 mmoles) of 3-mercapto-1H-1,2,4-triazole and 100 mg (0.25 mmole) of di-4-nitrophenyl N-(4-toluenesulphonyl)-phosporamidate in 200 ml of dichloromethane. The calculated amount of ammonia was evolved after refluxing for 1.25 hours. Refluxing was continued for 0.5 hour and then the clear solution obtained was evaporated to dryness, yielding 23.1 g (98%) of 1-trimethylsilyl-3-trimethylsilylthio-1H-1,2,4-triazole, m.p. 90-94°C.

PMR ($CCl_4$): 0.52 (s, 9H); 0.55 (s, 9H); 7.52 (s, 1H).

## Example 28

A mixture consisting of 2.85 g (25 mmoles) of 1-methyl-2-mercaptoimidazole, 22 mg (0.12 mmole) of saccharin, 20 ml of toluene and 5.2 ml (25 mmoles) of hexamethyldisilazane was refluxed for 1 hour. After cooling to room temperature 5.40 g (25 mmoles) of 4-nitrobenzyl bromide and then 5 ml of hexamethylphosphoric triamide were added to the mixture which contained 1-methyl-2-(trimethylsilylthio)imidazole. After stirring for 2 hours at room temperature, the mixture was diluted with ethyl acetate to 150 ml. The solution thus obtained was washed three times with 50 ml of a saturated sodium bicarbonate solution and then twice with 20 ml of water. The organic layer was dried, filtered and evaporated to dryness. The crystalline residue was washed with 100 ml of petroleum ether (boiling range 60-80°C) and then vacuum dried. There were obtained 5.54 g (89%) of 1-methyl-2-(4-nitrobenzylthio)imidazole, m.p. 74-77°C. Crystallization of a sample from ethanol raised the melting point to 77.5-78.0°C.

Example 29

A solution of 1-methyl-2-(trimethylsilylthio)imidazole was prepared by refluxing a mixture of 1.16 g (10 mmoles) of 1-methyl-2-mercaptoimidazole, 18 mg (0.1 mmole) of saccharin, 1.5 ml (7.2 mmoles) of hexamethyldisilazane and 25 ml of acetonitrile for one hour. Hexamethylphosphoric triamide (1.8 ml) and 4-nitrobenzyl bromide (2.3 g; 10.5 mmoles) were added and refluxing was continued for 10 minutes. The acetonitrile was evaporated and 100 ml of ethyl acetate were added to the residue. The crystals were filtered off and washed with ethyl acetate. A yield of 2.93 g (88.8%) of 1-methyl-2-(4-nitrobenzylthio)imidazole hydrogen bromide salt with m.p. 183-185°C was obtained. The filtrate was washed with water, dried and evaporated to dryness. Diethyl ether was added to the residue and the solid was filtered off. There was obtained 0.3 g (12%) of 1-methyl-3-(4-nitrobenzyl)-1,2-dihydroimidazole-2-thione as a by-product, m.p. 162- 168°C. Crystallization from a mixture of chloroform and carbon tetrachloride raised the melting point to 167-168°C.

PMR (DMSO-d6): 3.32 (s, 2H); 5.31 (s, 3H); 7.14 (s, 2H); 7.35, 7.49, 8.04 and 8.19 (4s, 4H);

IR: 3160, 3124, 3100, 3067, 1606, 1598, 1570, 1510, 1340 $cm^{-1}$.

Example 30

A suspension was made of 0.50 g of 7-phenylacetamido-3-bromomethyl-3-cephem-4-carboxylic acid 1-oxide with a content of 86% (1 mmole) in 10 ml of dichloromethane. Hexamethyldisilazane (0.20 ml, 0.96 mmole) was added and the mixture was stirred for 45 minutes. To the clear solution obtained 0.25 ml of hexamethylphosphoric triamide and 0.36 g (1.66 mmole) of 4-chlorophenylthio(trimethyl)silane were added subsequently. After stirring for 30 minutes at room temperature, the reaction was quenched by the addition of 2 ml of methanol, which resulted in the forming of a precipitate. The mixture was evaporated to dryness and then, 20 ml of diethyl ether were added. The solid was collected by filtration and

BAD ORIGINAL

washed with 10 ml of diethyl ether, 10 ml of 0.1 N HCl and twice with 10 ml of diethyl ether. The product was vacuum dried at room temperature. There was obtained 0.48 g of 7-phenylacetamido-3-(4-chlorophenyl)thiomethyl-3-cephem-4-carboxylic acid 1-oxide with a purity of 97% according to quantitative PMR analysis; yield 95%.

PMR (DMSO-D6): 3.49, 3.75, (ABq, 2H, J 15 Hz); 3.79, 4.39 (ABq, 2H, J 13.5 Hz); 3.84 (s, 2H); 4.87 (d, 1H, 4.5 Hz); 5.77 (dd, 1H, J 4.5 and 8 Hz); 7.30 (s, 4H); 7.38 (s, 5H); 8.39 (d, 1H, J 8 Hz).

IR: 3270, 1774, 1765, 1723, 1658, 1520, 1240, 1010, 997 $cm^{-1}$.

BAD ORIGINAL

## Claims

1. Process for the preparation of thioethers which comprises reacting a silylated thiol of the general formula:

$$R - S - SiR_1R_2R_3 \qquad I$$

wherein R represents an organic group and $R_1$, $R_2$ and $R_3$ are the same or different and each represents an alkyl group having 1 to 4 carbon atoms, with an organic halide, sulphate or sulphonate in the presence of hexamethylphosphoric triamide as a solvent or a co-solvent.

2. Process according to claim 1 in which the reaction is carried out under neutral conditions in aprotic solvents.

3. Process according to claim 1 in which the reaction is carried out at a temperature between 0° and 150°C.

4. Process according to claim 3 in which the reaction is carried out at a temperature between 20° and 80°C.

5. Process according to claim 1 in which the organic halide is a chloride, bromide or iodide.

6. Process according to claim 1 in which the organic group of the halide is a straight or branched-chain alkyl or alkenyl group, an aryl or aralkyl group or a heterocyclic group, each of which groups may be further substituted by one or more groups which do not interfere with the reaction defined in claim 1.

7. Process according to claim 1 in which the organic sulphate is a dialkyl sulphate.

8. Process according to claim 1 in which the organic sulphonate is an alkyl sulphonate.

9. Process according to claim 8 in which the alkyl sulphonate is an alkyl ester of benzene or naphthalene sulphonic

acid.

10. Process according to claim 1 in which the organic group R is an alkyl, aryl, aralkyl or heterocyclic group, each of which groups may be further substituted by one or more groups which do not interfere with the reaction defined in claim 1.

11. Process according to claim 10 in which the heterocyclic group is a 5- or 6-membered group having one or more nitrogen or sulphur atoms as the heteroatoms.

European Patent Office

# EUROPEAN SEARCH REPORT

0088831
Application number

EP 82 20 0327.3

## DOCUMENTS CONSIDERED TO BE RELEVANT

| Category | Citation of document with indication, where appropriate, of relevant passages | Relevant to claim |
|---|---|---|
| D,A | BULLETIN OF THE CHEMICAL SOCIETY OF JAPAN, Vol. 54, No. 5, 1981<br>Tokyo<br>S. KOZUKA et al. "Kinetic Study on the Reaction of (Arylthio)trimethylsilanes with Phenacyl Bromide Giving Aryl Phenacyl Sulfides and Bromotrimethylsilane"<br>pages 1420 to 1423<br>-- | 1 |
| D,A | JOURNAL OF THE CHEMICAL SOCIETY, 1964<br>Letchworth<br>E.W. ABEL et al. "The Reactions of Alkylthiotrimethylsilanes, Hexamethyldisilthiane, and Hexamethylcylotrisilthiane with Some Organic Halides"<br>pages 2455 to 2458<br>-- | 1 |
| D,A | JOURNAL OF THE CHEMICAL SOCIETY, 1960<br>Letchworth<br>E.W. ABEL et al. "The Alkylthiotrimethylsilanes: Preparations and Some Properties"<br>pages 4406 to 4409<br>---- | 1 |

X The present search report has been drawn up for all claims

| Place of search | Date of completion of the search | Examiner |
|---|---|---|
| Berlin | 20-07-1982 | PHILLIPS |

CLASSIFICATION OF THE APPLICATION (Int. Cl. 3)

C 07 C 148/00
C 07 C 149/30
C 07 D 501/36
C 07 D 285/12
C 07 D 257/04
C 07 C 149/31
C 07 C 149/34
C 07 C 149/40
C 07 C 149/415
C 07 C 153/11
C 07 D 233/84
C 07 D 249/12
C 07 D 311/56

TECHNICAL FIELDS SEARCHED (Int.Cl. 3)

C 07 C 148/00
C 07 C 149/00

CATEGORY OF CITED DOCUMENTS

X: particularly relevant if taken alone
Y: particularly relevant if combined with another document of the same category
A: technological background
O: non-written disclosure
P: intermediate document
T: theory or principle underlying the invention
E: earlier patent document, but published on, or after the filing date
D: document cited in the application
L: document cited for other reasons

&: member of the same patent family, corresponding document

EPO Form 1503.1 06.78